(19) Europäisches Patentamt
European Patent Office
Office européen des brevets

(11) **EP 2 491 883 B1**

(12) **EUROPÄISCHE PATENTSCHRIFT**

(45) Veröffentlichungstag und Bekanntmachung des Hinweises auf die Patenterteilung:
**18.06.2014 Patentblatt 2014/25**

(51) Int Cl.:
***A61B 18/14*** (2006.01)  ***G01L 1/24*** (2006.01)
***A61M 25/00*** (2006.01)

(21) Anmeldenummer: **12154649.3**

(22) Anmeldetag: **09.02.2012**

(54) **Katheter und Katheteranordnung**

Catheter and catheter arrangement

Cathéter et système de cathéter

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.02.2011 US 201161446053 P**

(43) Veröffentlichungstag der Anmeldung:
**29.08.2012 Patentblatt 2012/35**

(73) Patentinhaber: **VascoMed GmbH**
**79589 Binzen (DE)**

(72) Erfinder:
• **Fandrey, Stephan**
**8910 Affoltern am Albis (CH)**

• **Diebold, Michael**
**12169 Berlin (DE)**

(74) Vertreter: **Lindner-Vogt, Karin L.**
**Biotronik SE & Co. KG**
**Corporate Intellectual Properties**
**Woermannkehre 1**
**12359 Berlin (DE)**

(56) Entgegenhaltungen:
**WO-A1-2004/104539    WO-A1-2009/023801**
**WO-A2-2007/015139    WO-A2-2008/131303**
**WO-A2-2009/136311**

## Beschreibung

Die Erfindung betrifft ein Katheter, insbesondere Ablationskatheter, mit in einen distalen Abschnitt integrierten Kraftsensoren, die zur Messung des Betrages und der Richtung einer auf den distalen Abschnitt wirkenden äußeren Kraft ausgebildet und angeordnet und zur gemeinsamen Messsignalverarbeitung an eine Signalverarbeitungseinheit anschließbar sind.

In bestimmten Einsatzfeldern von Kathetern oder ähnlichen Vorrichtungen, z.B. Elektrodenleitungen, ist eine Andruckkraft an benachbartes Gewebe von Bedeutung für deren Funktion, sodass eine Erfassung dieser Kontaktkraft von Interesse ist. Dies trifft in besonderem Maße für sogenannte Ablationskatheter zu, mit denen Gewebebereiche verödet bzw. Gewebeteile abgetragen werden.

Es ist ein Ablationskatheter ("TactiCath", Fa. Endosense) bekannt, welches die Messung einer auf das distale Katheterende einwirkenden Kraft - im Anwendungsfall also der erwähnten Kontaktkraft - nach Betrag und Richtung während eines Ablationsvorganges ermöglicht. Dieser Katheter nutzt das Prinzip des sogenannten FBG(Faser-Bragg-Gitter)-Sensors, wobei drei Fasern mit jeweils einem FBG-Sensor am Faserende die für eine 3D-Kraftmessung benötigte Gruppe von Sensoren bilden, die zur gemeinsamen Messsignalverarbeitung an eine Signalverarbeitungseinheit einschließbar sind. Die Sensoren sind in einem Winkelabstand von 120° außen auf einem verformbaren Zylinder aufgebracht.

In der US 2008/0285909 A1 wird die Funktionsweise von FBG-Sensoren zur Bestimmung von Verdrillungen oder Krümmungen des Katheterkörpers ausführlich beschrieben, und auch die Funktionsweise des vorgenannten Kraftsensors mit mehreren FBG-Fasern auf einem verformbaren Zylinder ist in dieser Druckschrift erläutert.

Es ist, wie in der WO 2009/138957 A2 beschrieben, eine Temperaturkompensation mittels dreier elektrischer Thermoelemente vorgesehen, weil bei dem FBG-Messverfahren bereits kleine Temperaturänderungen bzw. Abweichungen zwischen den einzelnen Sensoren große Messunsicherheiten verursachen können und bei einem elektrothermischen Ablationsvorgang ganz erhebliche Temperaturschwankungen an der Spitze des Ablationskatheters auftreten können.

Das optische Messprinzip der FBG-Sensorik ist allgemein sowie insbesondere auch in seiner Anwendung für Kraftmessungen und Temperaturmessungen bekannt; vgl. etwa www.wikipedia.org/wiki/Fiber_Bragg_grating oder A. Othonos, K. Kalli:"Fiber Bragg Gratings: Fundamentels and Applications in Telecommunications and Sensing" Artec House 1999, sowie (speziell bezogen auf Spannungs- und Temperaturmessungen) US 5,399,854. Eine ausführliche Erläuterung des Messprinzips ist daher hier nicht erforderlich.

Unabhängig von diesem Messprinzip sind auch andere Lösungen für eine Kontaktkraftmessung an einem Führungsdraht oder Katheter bekannt, bspw. unter Einsatz eines optischen Sensors, wie in der WO 2009/007857 A2 beschrieben, oder unter Einsatz eines Halbleitersensors an der Spitze eines Führungsdrahtes, wie in der WO 2008/003307 A2 beschrieben.

Siehe desweiteren WO 2009/023801 A1, WO 2009/136311 A2 und WO 2008/131303 A2.

Der Erfindung liegt die Aufgabe zugrunde, einen sowohl in seinem Aufbau als auch im Bezug auf den Anschluss an eine zugehörige Signalverarbeitungseinheit vereinfachten und daher kostengünstigen Katheter der eingangs genannten Art anzugeben.

Diese Aufgabe wird durch einen Katheter mit den Merkmalen des Anspruchs 1 gelöst.

Die Erfindung schließt den wesentlichen Gedanken ein, in einem Katheter mit Kraftsensoren zur 3D-Kraftmessung eine einzelne FBG-Faser vorzusehen, welche die zur Erfassung von Betrag und Richtung der äußeren Kraft (speziell Kontaktkraft) erforderlichen Kraftsensorbereiche umfasst. Sie schließt des Weiteren den Gedanken ein, diese einzelne Faser in einem Sensorhalter in einer Weise zu fixieren, dass die Kraftsensorbereiche in/auf der Faser bestimmungsgemäß funktionieren können.

Insbesondere wird mit der Erfindung also ein einfach aufgebauter, in eine Katheterspitze integrierter Kontaktkraftsensor bereitgestellt, der speziell während einer Ablation die Kontaktkraft zwischen der Katheterspitze und der Gewebewand messen kann. Dies ermöglicht es letztlich, einen definierten Kontakt zwischen einer Ablationselektrode an der Katheterspitze und der Gefäßwand herzustellen, wodurch sich zuverlässig ein zufriedenstellender Erfolg des Ablationsvorganges erreichen lässt. Zudem kann mit der erfindungsgemäßen Lösung auch eine einfachere und kostengünstigere Signalverarbeitungseinheit verwendet werden, da die auf einer einzelnen Faser angeordneten Sensoren über einen einzigen Kanal ausgelesen werden können.

Insbesondere sind die drei Kraftsensorbereiche in Längsrichtung der FBG-Faser beabstandet auf dieser ausgebildet.

In einer Ausführung der Erfindung ist die FBG-Faser dicht an einem Kühlkanal des Katheters derart verlegt, dass die Kraftsensorbereiche im Betrieb eine Temperatur nahe der Temperatur einer durch den Kühl- oder Spülkanal geleiteten Kühl- oder Spülflüssigkeit annehmen. Mit dieser Ausführung wird eine Temperaturstabilisierung der Sensorbereiche bezweckt, so dass nur ein geringer Einfluss von äußeren Temperaturänderungen (bspw. während eines Ablationsvorganges) auf die Sensorik zu verzeichnen ist und ggf. zusätzliche Elemente zur Temperaturmessung und -kompensation entfallen können.

In einer Ausgestaltung dieser Ausführung ist der Spülkanal mindestens in einem Abschnitt des Katheters, in dem auf der FBG-Faser die Kraftsensorbereiche ausgebildet sind, als Helix geformt und die FBG-Faser in oder nahe der Längsachse der Helix verlegt.

In einer weiteren Ausgestaltung ist im Spülkanal ein Temperatursensor angeordnet, um einen der tatsächlichen Temperatur der Sensorbereiche sehr nahe kommenden Temperatur-Bezugswert verfügbar zu haben. Dieser Sensor ermöglicht es nämlich, dass die Temperatur durch die Spülflüssigkeit über die Länge der Fasergitter (Sensorbereiche) zur Kraftmessung bekannt ist und herausgerechnet bzw. durch eine katheter-spezifische KalibrierungsMatrix eine T-Kompensation vorgenommen werden kann.

Im Sinne der vorstehend erläuterten Ausführung und Ausgestaltungen ist es auch, die FBG-Faser soweit als möglich zentriert mit einem großen Abstand zur Katheter-Außenwand im Katheterkörper unterzubringen.

In einer weiteren Ausführung sind auf der FBG-Faser nahe den Kraftsensorbereichen, insbesondere zwischen diesen, Temperatursensorbereiche ausgebildet. Dies ermöglicht insbesondere bei Anwendungen mit hohen Temperaturschwankungen bzw. konstruktiven Ausgestaltungen, wo durch die Spülflüssigkeit keine ausweichende Temperaturkonstanz der Kraftsensorbereiche erreicht werden kann, eine rechnerische Temperaturkompensation der Signale der einzelnen Kraftsensorbereiche. Die Temperatursensorbereiche sind in dem weiter oben erwähnten Sensorhalter insbesondere mechanisch zu entlasten, um Verfälschungen der T-Signale durch mechanische Belastungen der Faser zu vermeiden.

In einer weiteren Ausführung ist am distalen Ende der FBG-Faser ein Temperatursensorbereich ausgebildet. Eine Temperaturmessung am Katheterende ist insbesondere bei Ablationskathetern gewünscht, und diese kann bei der erwähnten Ausführung zusammen mit der Kraftmessung einer einzelnen Faser realisiert werden. Dies wiederum ermöglicht eine einfachere Ausführung der zugehörigen Signalverarbeitungseinheit und des Anschlusses an diese.

Im Sinne dieser Vereinfachungen ist in einer weiteren Ausführung vorgesehen, dass die einzelnen Kraftsensorbereiche und insbesondere auch die optional vorgesehenen Temperatursensorbereiche zum Betrieb mit verschiedenen Wellenlängen derart ausgelegt sind, dass ein Auslesen über einen einzigen Eingangskanal der Signalverarbeitungseinheit möglich ist.

Der Sensorhalter des erfinderischen Katheters weist korrespondierend zur Position der Kraftsensorbereiche konstruktiv vorbestimmte mechanische Schwachstellen, insbesondere in Form einer helixförmig verlaufenden Ausnehmung oder mehrerer kreissegmentförmiger Ausnehmungen in einem zylindrischen Haltergehäuse, auf. Hiermit wird die FBG-Faser einerseits mechanisch geschützt, und es ist die erforderliche mechanische Entlastung der optional vorgesehenen Temperatursensorbereiche hierdurch möglich. Andererseits gewährleistet dieser Aufbau, dass die grundsätzlich als Dehnungsmessbereiche wirkenden Kraftsensorbereiche ihre bestimmungsgemäße Funktion entfalten können.

Im Hinblick darauf, dass eine hochgenaue Kraftmessung die Kenntnis sensorspezifischer bzw. katheterspezifischer Daten des einzelnen Produkts in einer eingeschlossenen Auswertungseinheit erfordert, hat der Katheter gemäß einer weiteren Ausführung der Erfindung einen, insbesondere am oder nahe einem proximalen Ende angeordneten, Informationsträger mit spezifischer Kalibrierungsinformation. In einer Ausgestaltung ist der Informationsträger als EEPROM, RFID-Tag oder Barcode ausgebildet, welcher mit an sich bekannten und kostengünstigen Mitteln an der Signalverarbeitungseinheit leicht und zuverlässig ausgelesen werden kann.

In einer Ausführung der vorgeschlagenen Katheteranordnung ist vorgesehen, dass die Signalverarbeitungseinheit einen Eingangskanal für Signale von Temperatursensorbereichen und/oder eines Temperatursensors im Spülkanal des Katheters und eine Temperatur-Berechnungseinheit und/oder Kompensations-Verarbeitungsmittel zur TemperaturKompensation der Signale der Kraftsensoren umfasst. Insbesondere ist ein gemeinsamer Eingangskanal für Signale der Kraftsensoren und der Temperatursensorbereiche vorgesehen.

In einer weiteren Ausgestaltung der Anordnung ist vorgesehen, dass die Signalverarbeitungseinheit Auslesemittel zum Auslesen von Kalibrierungsinformation von einem Informationsträger am Katheter aufweist.

Ausgestaltungen der vorgeschlagenen Kraftmesseinrichtung ergeben sich im Prinzip aus den weiter oben erläuterten Ausgestaltungen des vorgeschlagenen Katheters,

Vorteile und Zweckmäßigkeiten der Erfindung ergeben sich im Übrigen aus den nachfolgenden Beschreibung von Ausführungsbeispielen anhand der Figuren. Von diesen zeigen:

Fig. 1 eine Prinzipskizze des Aufbaus einer FBG-Faser,

Fig. 2 eine Prinzipskizze der Sensorkonstruktion bei einer Ausführungsform des erfindungsgemäßen Katheters,

Fig. 3 eine Prinzipskizze einer Ausführungsform eines exemplarischen Katheters mit speziell gestaltetem Spülkanal und

Fig. 4A und 4B Prinzipskizzen des Aufbaus der Sensorhalter bei zwei weiteren Ausführungsformen des erfindungsgemäßen Katheters.

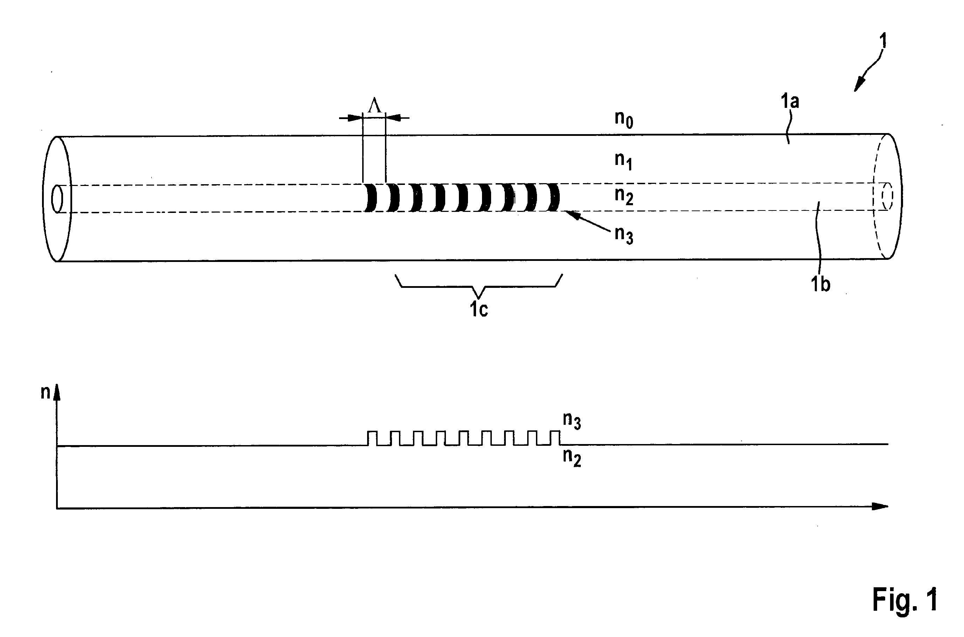

Fig. 1 zeigt im oberen Teil als schematische perspektivische Darstellung einen Abschnitt einer FBG-Faser 1, die eingebetet in einen Mantel 1a mit einem Brechungsindex $n_1$, einen Kern 1b hat, dessen Brechungsindex im Wesentlichen über die gesamte Längserstreckung $n_2$ ist. In einem Sensorabschnitt 1c der Faser 1 ist ein Bragg-Gitter ausgebildet, in dem Abschnitte mit dem Brechungsindex $n_2$ sich mit Abschnitten eines anderen Brechungsindex $n_3$ abwechseln, wie im unteren Teil der Figur verdeutlicht. Der Sensorabschnitt $1_c$ der Faser 1 kann unter anderem zur Erfassung von Spannungen bzw. äußeren Krafteinwirkungen oder Temperaturänderungen genutzt werden, wie aus dem Stand der Technik bekannt ist und daher hier nicht genauer beschrieben wird.

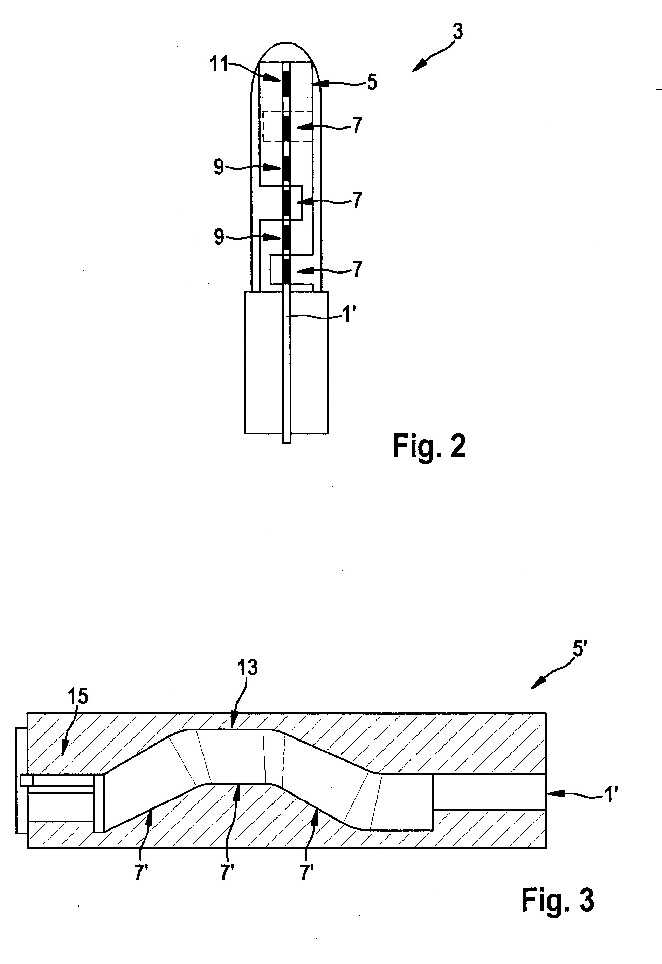

Fig. 2 zeigt als Prinzipskizze einen distalen Abschnitt eines Ablationskatheters 3, in dessen Zentrum eine FBG-Faser 1 verläuft und in einem Sensorhalter 5 gehalten ist. In der FBG-Faser 1' sind mehrere Sensorbereiche mit unterschiedlicher Funktion realisiert, und zwar drei Kraft bzw. Dehnungssensorbereiche 7, zwei zwischen diesen liegende Temperatursensorbereiche 9, deren Signale zur T-Kompensation der Signale der Kraftsensorbereiche 5 verwendet werden, und am distalen Ende der Faser 1' ein weiterer Temperatursensorbereich 11 zur T-Bestimmung der Elektrodenspitze im Einsatz, also bei einem Ablationsvorgang. Die einzelnen FBG-Sensorbereiche 7, 9 und 11 lassen sich so herstellen, dass jeder auf der Faser 1' (über die prinzipbedingt zugleich die Signale übertragen werden) zur Nutzung einer anderen Wellenlänge ausgebildet ist. Damit kann jeder Sensorbereich unabhängig ausgelesen werden.

Die zwischen den Kraftsensorbereich vorgesehenen Temperatursensorbereiche liefern Signale, die im Falle einer nicht ausreichenden Temperaturstabilisierung des distalen Katheterabschnitts für eine T-Kalibrierung benutzt werden können.

Weiterhin muss zur Berechnung eines 3D-Kraftvektors eine Kalibrierung des Sensorsystems stattfinden indem der Sensor nacheinander mit drei zueinander senkrechten Kräften (Fx, Fy, Fz) belastet wird.

In der Theorie berechnen sich die Dehnungen $\varepsilon_1$, $\varepsilon_2$ und $\varepsilon_3$ in den verteilten Sensorbereichen mit den Biegesteifigkeiten EI, den Biegemomenten $Fl$ und den Abstand $r$ zur dehnungsneutralen Faser wie folgt (Fig. 1):

$$\varepsilon_1 = \frac{F_x \cdot l_1}{E \cdot I_{xx1}} \cdot r_{11} + \frac{F_y \cdot l_1}{E \cdot I_{yy1}} \cdot r_{12} + \frac{F_z}{A \cdot E}$$

$$\varepsilon_2 = \frac{F_x \cdot l_2}{E \cdot I_{xx2}} \cdot r_{21} + \frac{F_y \cdot l_2}{E \cdot I_{yy2}} \cdot r_{22} + \frac{F_z}{A \cdot E}$$

$$\varepsilon_3 = \frac{F_x \cdot l_3}{E \cdot I_{xx3}} \cdot r_{31} + \frac{F_y \cdot l_3}{E \cdot I_{yy3}} \cdot r_{32} + \frac{F_z}{A \cdot E}$$

Mit drei Messungen, in denen drei zueinander senkrechte Kräfte verwendet werden, kann eine entsprechende Kalibrierungsmatrix berechnet werden:

$$\begin{pmatrix} C_{11} & C_{12} & C_{13} \\ C_{21} & C_{22} & C_{23} \\ C_{31} & C_{32} & C_{33} \end{pmatrix} = \begin{pmatrix} F_x & 0 & 0 \\ 0 & F_y & 0 \\ 0 & 0 & F_z \end{pmatrix} \cdot \begin{pmatrix} \varepsilon_{11} & \varepsilon_{12} & \varepsilon_{13} \\ \varepsilon_{21} & \varepsilon_{22} & \varepsilon_{23} \\ \varepsilon_{31} & \varepsilon_{32} & \varepsilon_{33} \end{pmatrix}^{-1} \qquad \text{Eq. 2}$$

Dabei müssen die Biegesteifigkeiten $EI$ und Abstände zur neutralen Faser $r$ in dem Sensorhalter in der Art konstruiert werden, dass sich drei linear unabhängige Gleichungen ergeben. So ist es möglich einen 3D-Kraftvektor aus gemessenen Dehnungen einer beliebigen Kraftrichtung mit Hilfe der Kalibrierungsmatrix zu berechnen:

$$\begin{pmatrix} F_x \\ F_y \\ F_z \end{pmatrix} = \begin{pmatrix} C_{11} & C_{12} & C_{13} \\ C_{21} & C_{22} & C_{23} \\ C_{31} & C_{32} & C_{33} \end{pmatrix} \cdot \begin{pmatrix} \varepsilon_1 \\ \varepsilon_2 \\ \varepsilon_3 \end{pmatrix} \qquad \text{Eq. 3}$$

Diese Kalibrierungsdaten werden optional im Katheter auf einem EEPROM, RFID oder einem 2D-Barcode gespeichert und von der Auswerteeinheit drahtlos, drahtgebunden oder beim 2D-Barcode optisch ausgelesen.

Fig. 3 zeigt einen Sensorhalter 5' eines exemplarischen Katheters, bei dem ein Spülkanal 13 im distalen Abschnitt, wo drei Kraftsensorbereiche 7' auf der FBG-Faser 1' angeordnet sind, helixförmig die Faser umgebend ausgebildet ist. Der Faserkanal 15 als solcher ist hierbei geradlinig ausgeführt. Die helixartige Ausbildung des Spülkanals 13 um den Faserkanal 15 herum soll für die Einstellung einer relativ konstanten Temperatur der Sensorbereiche 7' auch bei Anwendungen mit stark schwankenden Temperaturen (wie etwa einer Ablation) sorgen. Zudem sorgt die spezielle Ausführung des helixartigen Spülkanals für die lineare Unabhängigkeit der drei Kraftsensorbereiche.

Tabelle 1 zeigt Ergebnisse einer gemäß dem oben skizzierten Algorithmus ausgeführten Simulationsrechnung für den Sensorhalter 5', aus denen sich dessen Funktionsfähigkeit herleiten lässt.

Tabelle 1: Beispielergebnis aus einer Simulation des Sensorhalters nach Fig. 3

| In der Simulation eingeleitete Kraft | Simulierte Dehnung der drei Sensorbereiche | Berechneter Kraftvektor aus den simulierten Dehnungen |
|---|---|---|
| Fx= -0.25 N <br> Fy= -0.25 N <br> Fz= 0 N | $\varepsilon_1$ 96 $\mu$strain <br> $\varepsilon_2$= 116 $\mu$strain <br> $\varepsilon_3$= 64 $\mu$strain | Fx= -0.2602 N <br> Fy= -0.255 N <br> Fz= -0.006 N |

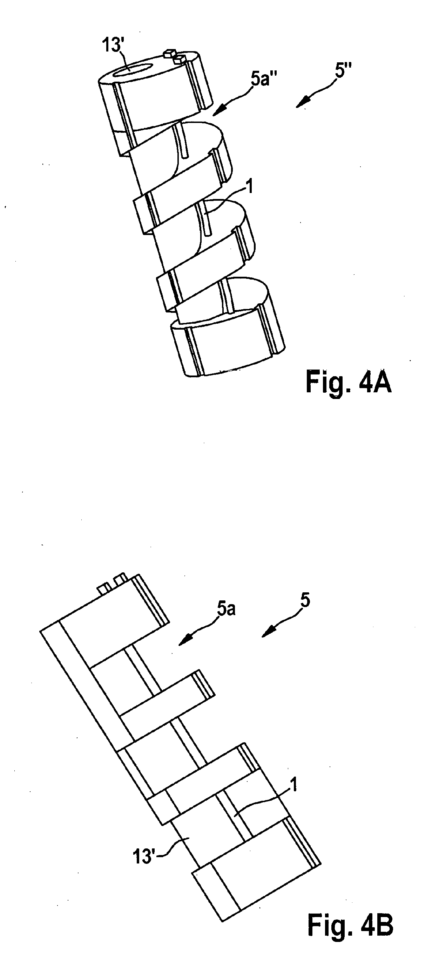

Fig. 4A zeigt einen weiteren Sensorhalter 5", der neben einem - hier geradlinig lang gestreckt verlaufende - Spülkanal 13', die ebenfalls geradlinig langgestreckt verlaufende FBG-Faser 1 hält und eine helixförmig verlaufende Ausnehmung 5a" als Schwächungsbereich aufweist. Durch diesen wird eine weitgehend freie Krafteinwirkung auf die (hier nicht dargestellt) Kraftsensorbereiche auf der Faser 1 sichergestellt.

Tabelle 2 zeigt Beispielergebnisse einer Simulationsrechnung für diesen Sensorhalter 5".

| In der Simulation eingeleitete Kraft | Simulierte Dehnung der drei Sensorbereiche | Berechneter Kraftvektor aus den simulierten Dehnungen |
|---|---|---|
| Fx=0.35N <br> Fy= -0.125 N <br> Fz=-0.2N | $\varepsilon_1$= 59 $\mu$strain <br> $\varepsilon_2$= 210 $\mu$strain <br> $\varepsilon_3$=406 $\mu$strain | Fx= 0.3484 N <br> Fy= -0.120 N <br> Fz= -0.1919 N |

Fig. 4B zeigt als weitere Ausführungsform den Sensorhalter 5 in perspektivischer Darstellung, der skizzenartig bereits in Fig. 2 zu erkennen ist. Auch in diesem Verlaufen der Spülkanal 13' und die FBG-Faser 1 dicht benachbart und geradlinig langgestreckt, die Schwächungsbereiche 5a sind hier voneinander separierte Ausnehmungen um 120° versetzt in der Form von Kreisringsegmenten ausgebildet, um auch hier wieder die lineare Unabhängigkeit der Sensoren zu erreichen.

Tabelle 3 zeigt Simulationsergebnisse für diesen Sensorhalter 5.

| In der Simulation eingeleitete Kraft | Simulierte Dehnung der drei Sensorbereiche | Berechneter Kraftvektor aus den simulierten Dehnungen |
|---|---|---|
| Fx= 0.35 N <br> Fy= -0.125 N <br> Fz= -0.2 N | $\varepsilon_1$= 37 $\mu$strain <br> $\varepsilon_2$= 224 $\mu$strain <br> $\varepsilon_3$= 281 $\mu$strain | Fx= 0.35002 N <br> Fy= -0.1249 N <br> Fz= -0.19997 N |

Die Ausführung der Erfindung ist nicht auf dir oben beschriebenen Beispiele und hervorgehobenen Aspekte beschränkt, sondern kann ebenso in einer Vielzahl von Abwandlungen erfolgen, die im Rahmen fachmännischen Handelns liegen.

**Patentansprüche**

1. Katheter, insbesondere Ablationskatheter, mit in einen distalen Abschnitt integrierten Kraftsensoren, die zur Messung des Betrages und der Richtung einer auf den distalen Abschnitt wirkenden äußeren Kraft ausgebildet und angeordnet und zur gemeinsamen Messsignalverarbeitung an eine Signalverarbeitungseinheit anschließbar sind, wobei in dem Katheter eine in einem Sensorhalter (5") fixierte einzelne FBG-Faser (1) vorgesehen ist, welche drei Kraftsensorbereiche (7, 7') umfasst, die die zur gemeinsamen Messsignalverarbeitung zusammenwirkenden Kraftsensoren bildet **gekennzeichnet dadurch, dass** der Sensorhalter korrespondierend zur Position der Kraftsensorbereiche konstruktiv vorbestimmte mechanische Schwachstellen (5a, 5a"), insbesondere in Form einer helixförmig verlaufenden Ausnehmung oder mehrerer kreissegmentförmiger Ausnehmungen in einem zylindrischen Haltergehäuse, aufweist.

2. Katheter nach Anspruch 1, wobei die drei Kraftsensorbereiche in Längsrichtung der FBG-Faser beabstandet auf dieser ausgebildet sind.

3. Katheter nach Anspruch 1 oder 2, wobei auf der FBG-Faser nahe den Kraftsensorbereichen, insbesondere zwischen diesen, Temperatursensorbereiche (9) ausgebildet sind.

4. Katheter nach einem der vorangehenden Ansprüche, wobei am distalen Ende der FBG-Faser ein Temperatursensorbereich (11) ausgebildet ist.

5. Katheter nach einem der vorangehenden Ansprüche, wobei die FBG-Faser dicht an einem Kühlkanal des Katheters verlegt ist, derart, dass die Kraftsensorbereiche im Betrieb eine Temperatur nahe der Temperatur einer durch den Kühlkanal geleiteten Spülflüssigkeit annehmen.

6. Katheter nach Anspruch 5, wobei der Kühlkanal mindestens in einem Abschnitt des Katheters, in dem auf der FBG-Faser die Kraftsensorbereiche ausgebildet sind, als Helix geformt und die FBG-Faser in oder nahe der Längsachse der Helix verlegt ist.

7. Katheter nach Anspruch 5 oder 6, wobei im Kühlkanal ein Temperatursensor angeordnet ist.

8. Katheter nach einem der vorangehenden Ansprüche, wobei die einzelnen Kraftsensorbereiche und insbesondere auch die optional vorgesehenen Temperatursensorbereiche zum Betrieb mit verschiedenen Wellenlängen ausgelegt sind, derart, dass ein Auslesen über einen einzigen Eingangskanal der Signalverarbeitungseinheit möglich ist.

9. Katheter nach einem der vorangehenden Ansprüche, mit einem, insbesondere am oder nahe einem proximalen Ende angeordneten, Informationsträger mit spezifischer Kalibrierungsinformation.

10. Katheter nach Anspruch 9 , wobei der Informationsträger als EEPROM, RFID-Tag oder Barcode ausgebildet ist.


**Claims**

1. A catheter, in particular ablation catheter, comprising force sensors, which are integrated in a distal portion, are configured and arranged to measure the magnitude and the direction of an external force acting on the distal portion, and can be connected to a signal processing unit for the combined processing of measurement signals, wherein an individual FBG fibre (1) fixed in a sensor holder (5") is provided in the catheter and comprises three force sensor regions (7, 7'), thus forming the force sensors cooperating for the combined processing of measurement signals, **characterised in that** the sensor holder has structurally predetermined mechanical weak points (5d, 5d") corresponding to the position of the force sensor regions, in particular in the form of a helically extending recess or a plurality of recesses shaped in the manner of segments of a circle in a cylindrical holder housing.

2. The catheter according to claim 1, wherein the three force sensor regions are distanced in the longitudinal direction of the FBG fibre and are formed thereon.

3. The catheter according to claim 1 or 2, wherein temperature sensor regions (9) are formed on the FBG fibre in the vicinity of the force sensor regions, in particular between these regions.

4. The catheter according to one of the preceding claims, wherein a temperature sensor region (11) is formed at the distal end of the FBG fibre.

5. The catheter according to one of the preceding claims, wherein the FBG fibre is laid closely against a cooling channel of the catheter in such a way that, during operation, the force sensor regions assume a temperature close to the temperature of a rinsing fluid conveyed through the cooling channel.

6. The catheter according to claim 5, wherein the cooling channel is formed as a helix at least in a portion of the catheter in which the force sensor regions are formed on the FBG fibre, and the FBG fibre is laid in or in the vicinity of the longitudinal axis of the helix.

7. The catheter according to claim 5 or 6, wherein a temperature sensor is arranged in the cooling channel.

8. The catheter according to one of the preceding claims, wherein the individual force sensor regions and in particular also the optionally provided temperature sensor regions are designed for operation with different wavelengths in such a way that a readout is possible via a single input channel of the signal processing unit.

9. The catheter according to one of the preceding claims, comprising an information carrier, in particular arranged at or in the vicinity of a proximal end, having specific calibration information.

10. The catheter according to claim 9, wherein the information carrier is formed as an EEPROM, RFID tag or barcode.


**Revendications**

1. Cathéter, en particulier un cathéter d'ablation avec des capteurs de force intégrés dans une section distale, lesquels sont conçus et agencés pour mesurer l'intensité et la direction d'une force extérieure agissant sur la section distale et peuvent être raccordés à une unité de traitement de signaux pour le traitement commun de signaux de mesure, dans lequel il est prévu une fibre FBG (1) individuelle fixée dans un support de capteurs (5") et comprenant trois régions de capteurs de force (7, 7'), lesquelles forment les capteurs de force coopérant pour le traitement commun des signaux de mesure, **caractérisé en ce que** le support de capteurs comporte des points faibles mécaniques (5a, 5a") prédéfinis dans la construction, correspondant aux emplacements des régions de capteurs de force, en particulier sous la forme d'un évidement s'étendant en forme d'hélice ou de plusieurs évidements en forme de segments de cercle dans un logement de support cylindrique.

2. Cathéter selon la revendication 1, dans lequel les trois régions de capteurs de force sont formées sur la fibre FBG et espacées les unes des autres dans le sens longitudinal de celle-ci.

3. Cathéter selon la revendication 1 ou 2, dans lequel des régions de capteurs de température (9) sont formées sur la fibre FBG, à proximité des régions de capteurs de force, en particulier entre celles-ci.

4. Cathéter selon l'une des revendications précédentes, dans lequel une région de capteurs de température (11) est formée à l'extrémité distale de la fibre FBG.

5. Cathéter selon l'une des revendications précédentes, dans lequel la fibre FBG est disposée serrée contre un canal de refroidissement, de manière à ce que pendant le fonctionnement, les régions de capteurs de force adoptent une température proche de la température d'un liquide de rinçage guidé à travers le canal de refroidissement.

6. Cathéter selon la revendication 5, dans lequel le canal de refroidissement est conçu comme une hélice au moins dans une section du cathéter, dans laquelle les régions de capteurs de force sont formées sur la fibre FBG, et la fibre FBG est disposée dans ou à proximité de l'axe longitudinal de l'hélice.

7. Cathéter selon la revendication 5 ou 6, dans lequel un capteur de température est agencé dans le canal de refroidissement.

8. Cathéter selon l'une des revendications précédentes, dans lequel les différentes régions de capteurs de force et en particulier également les éventuelles régions de capteurs de température sont conçues pour fonctionner avec différentes longueurs d'ondes, de manière à permettre une lecture par un seul canal d'entrée de l'unité de traitement de signaux.

9. Cathéter selon l'une des revendications précédentes, avec un support d'information avec une information de calibrage spécifique, agencé en particulier à une extrémité proximale ou à proximité de celle-ci.

10. Cathéter selon la revendication 9, dans lequel le support d'information est conçu comme un EEPROM, une étiquette RFID ou un code barres.

Fig. 1

**Fig. 2**

**Fig. 3**

13'

5a"

5"

1

**Fig. 4A**

5a

5

1

13'

**Fig. 4B**

**IN DER BESCHREIBUNG AUFGEFÜHRTE DOKUMENTE**

**In der Beschreibung aufgeführte Patentdokumente**

- US 20080285909 A1
- WO 2009138957 A2
- US 5399854 A
- WO 2009007857 A2
- WO 2008003307 A2
- WO 2009023801 A1
- WO 2009136311 A2
- WO 2008131303 A2

**In der Beschreibung aufgeführte Nicht-Patentliteratur**

- **A. OTHONOS ; K. KALLI.** Fiber Bragg Gratings: Fundamentels and Applications in Telecommunications and Sensing. Artec House, 1999